# EUROPEAN PATENT SPECIFICATION

(11) **EP 1 713 476 B1**
(45) Date of publication and mention of the grant of the patent: **05.05.2010**
(21) Application number: 04820975.3
(22) Date of filing: 03.11.2004
(51) Int. Cl.: A61K 31/4184

(54) **PROCESS FOR THE PREPARATION OF BENZIMIDAZOLES**
VERFAHREN ZUR HERSTELLUNG VON BENZIMIDAZOLEN
PROCEDE POUR LA PREPARATION DE BENZIMIDAZOLES

(30) Priority: 05.11.2003 IN MU11642003
(43) Date of publication of application: 25.10.2006
(73) Proprietor: Lyka Labs Limited, Mumbai - 400 099, Maharashtra (IN)
(72) Inventor: BAJAJ, Mannalal Ramgopal, Lyka Labs Limited, Mumbai - 400 099, Maharashtra (IN); SAMANT, Rajan Shantaram, Lyka Labs Limited, Mumbai - 400 099, Maharashtra (IN); SHAH, Bharat Babulal, Lyka Labs Limited, Mumbai - 400 099, Maharashtra (IN)
(74) Representative: Brand, Thomas Louis
(86) International application number: PCT/IN2004/000342
(87) International publication number: WO 2005/065682

(56) References cited:
- EP-A1- 1 310 252
- WO-A1-94/02414

## Description

### Related Application

This application claims priority from India National patent application serial No. 1164/MUM/2003, filed 5th November 2004.

### Technical Field of the Invention:

This invention relates to a process for the preparation of a novel drug delivery system for proton pump inhibitors (PPIs). More particularly, this invention relates to a process for the preparation of a stable, pharmaceutically acceptable, lyophilized injectable form of Rabeprazole.

### Background and Prior Art:

Proton pump inhibitors(PPIs) form the emerging anti-ulcer compounds and have already overtaken H2 antagonists like Ranitidine. PPIs are now the drugs of choice for stomach and duodenal ulcers. They are also effectively used to relieve symptoms of esophagitis and acute gastro-esophageal efflux. PPIs are also used to alleviate Helicobacter pylori infection which is considered to be the root cause of stomach ulcers. PPI's block the production of stomach acids by inhibiting a system in the stomach known as proton pump, also referred to as hydrogen-potassium adenosine triphosphate enzyme system.

Omeprazole (also esomeprazole), Lansoprazole, Pantoprazole and Rabeprazole are the leading commonly used proton pump inhibitors (PPIs). Owing to the close similarity between these PPIs, the formulations and dosage forms can be similarly formulated for the entire group of compounds based on a process developed for any one of the group of PPIs.

Rabeprazole (marketed as Aciphex in USA and other countries) is available only in tablet form or as delayed release tablets in NDDS.

Rabeprazole is currently administered by employing any suitable route of administration such as rectal, transdermal and like forms with effective dosage of active ingredient; however oral administration has hitherto been the preferred route. Reported oral dosage forms are tablets, troches, dispersions, suspensions, solutions, capsules and the like.

International Patent application WO9601624 describes a pharmaceutical formulation in the dosage form of multiple unit tablets which contains active ingredient, an acid labile H+K+ATPase inhibitor like Rabeprazole, or alkaline salts thereof.

Oral dosage forms for Rabeprazole are also disclosed in US patent number 5,035,899 and International Patent application WO97/12580 and WO97/25030.

Compositions of Rabeprazole suitable for rectal administration are described in European Patent 645140.

Further, the injections for PPI's have recently been developed. Japanese Patent unexamined Publication no. JP167587/1984 describes the process for preparation of injection of Omeprazole. The process comprises dissolving sodium salt of Omeprazole in sterilized water, filtering and lyophilizing the solution to give lyophilized product. This lyophilized product is dissolved in a mixture of polyethylene glycol 400 for injection, sodium dihydrogen phosphate and sterilized water.

For Lansoprazole the lyophilized injection is prepared by dissolving lyophilized product of Lansoprazole in a mixture of acid and at least one of ethanol, propylene glycol and polyethylene glycol as described in Japanese unexamined patent no. JP138213/1990.

Freeze dried injectable formulation of Pantoprazole is described in International Patent application WO0241919. Lyophilization of the aqueous solutions of Pantoprazole, ethylenediamine tetra acetic acid and/or a suitable salt thereof, and sodium hydroxide and/or sodium carbonate is disclosed.

Freeze dried formulations for Omeprazole and Lansoprazole, as described in International Patent application W09402141, comprise the benzimidazole compounds or their salts to which is added an aqueous solvent wherein the pH is not less than 9.5 and not more than 11.5.

An injectable composition is disclosed in EP1310252 comprising a benzimidazole compound having an antiulcer action and a strong alkali (e.g., an alkali metal hydroxide such as sodium hydroxide).

However, there is no formulation or delivery system for Rabeprazole, in particular, in injectable form. We have developed lyophilized, stable injectable dosage form of Rabeprazole, the process of which could also be applied for other PPIs like, Omeprazole, Lansoprazole, Pantoprazole, etc.

### Objective of the Invention:

The objective of the present invention is to provide a stabilized pharmaceutically acceptable dosage form of proton pump inhibitors and in particular stabilized lyophilized (freeze dried) injection of Rabeprazole.

### Summary of the Invention:

According to the present invention there is provided a process for the preparation of a stabilised drug delivery system which comprises dissolving sodium hydroxide in water suitable for injection and adjusting the pH of the solution above 12.0, adding mannitol and a benzimidazole compound to the above said solution whilst maintaining the pH of the said solution; making up the solution to a desired volume with water suitable for injection and lyophilizing the said made up solution to obtain in powder form the drug delivery system which is reconstitutable in a parenterally acceptable solvent to form an injectable solution, whilst maintaining the solution at a temperature of 10°C ± 2°C throughout the process prior to lyophilisation.

Said powder form, containing (a) Rabeprazole or its salts in a therapeutically effective total amount constituting about 8% to about 77% by weight and (b) mannitol 19% to 88%, by weight, of the composition.

Particularly the process for preparing a stabilized drug delivery system provides said composition which is reconstitutable in a parenterally acceptable solvent liquid, preferably an aqueous liquid, to form an injectable solution.

The said composition is prepared by a process which comprises lyophilization of an aqueous solution comprising Rabeprazole, mannitol, alkaline compounds and optionally other excipients to form a readily reconstitutable powder.

The present invention provides a process for preparing a novel drug delivery system for proton pump inhibitors which comprises, reconstituting a unit dosage of the composition in a physiologically acceptable volume of a parenterally acceptable solvent liquid, to form an injectable solution.

The said salts of Rabeprazole may be in the form of alkaline metal salts.or alkaline earth metal salts. The said alkaline metal salts may be sodium or potassium and the said alkaline earth metal salts may be calcium or magnesium.

The said system comprises Rabeprazole sodium, mannitol and alkaline compounds in the form of a stabilized lyophilized injection. The said alkaline compound is preferably sodium hydroxide. The pH of the said system after reconstitution is between 9-11.

Further the present invention provides a process for preparation of the said drug delivery system which comprises dissolving sodium hydroxide in Water for injection to adjust the pH above 12.0, adding Mannitol and Rabeprazole sodium to the above said solution; maintaining the pH the same; making up the volume with water for injection; filtering the said solution aseptically through 0.2211 filter paper ; and filling the said filtered solution in previously sterilized 10ml vial, after partial bunging; loading the vials into a lyophilizer and lyophilizing the said solution to obtain the said drug delivery system.

The process of stabilization and pharmaceutical excipients or ingredients used therein provides unique and novel stability and efficacy to the composition.

The term, "Rabeprazole" is not intended to be limited only to Rabeprazole, but is intended to include all benzimidazole compounds and their pharmaceutically acceptable salts.

### Detailed Description:

The present invention provides a process for preparing a pharmaceutical composition comprising in powder form, a stabilized drug delivery system in accordance with the following claims. Thus the present invention provides a process for preparing a pharmaceutical composition comprising dissolving sodium hydroxide in water suitable for injection and adjusting the pH of the solution above 12.0, adding mannitol and a benzimidazole compound to the above solution whilst maintaining the pH of the said solution; making up the solution to a desired volume with water suitable for injection and lyophilizing the said made up solution to obtain in power from the drug delivery system which is reconstitutable in a parenterally accepted solvent to form an injectable solution.

The process for preparing a pharmaceutical composition comprising in powder form, (a) Rabeprazole or its salts in a therapeutically effective total amount constituting about 8% to about 77% by weight and (b) mannitol 19% to 88%, by weight, of the composition.

Particularly the said composition is reconstitutable in a parenterally acceptable solvent, preferably an aqueous liquid, to form an injectable solution.

Stability and efficacy are incorporated into the composition by the novel process and pharmaceutical excipients and ingredients employed.

A process for preparing a composition which comprises lyophilization of an aqueous solution containing Rabeprazole, mannitol, alkaline compounds and optionally other excipients to form a readily reconstitutable powder.

The present invention provides a process for preparing a novel drug delivery system for proton pump inhibitors which comprises, reconstituting a unit dosage of the composition in a physiologically acceptable volume of a parenterally acceptable solvent liquid to form an injectable solution.

The said salts of Rabeprazole may be in the form of alkaline metal salts or alkaline earth metal salts. The said alkaline metal salts may be sodium or potassium and the said alkaline earth metal salts may be calcium or magnesium.

The said system comprises Rabeprazole sodium, mannitol and alkaline compounds in the form of stabilized lyophilized injection.

The said alkaline compound is preferably sodium hydroxide. The pH of the said system after reconstitution is between 9-11.

Further the present invention provides a process for preparation of the said drug delivery system comprising dissolving sodium hydroxide in Water for injection to adjust the pH above 12.0, adding Mannitol and Rabeprazole sodium to the said above solution; maintaining the pH the same; making up the volume with water for injection; filtering the said solution aseptically through 0. 22u filter paper and filling the said filtered solution in previously sterilized 10ml vials; the solution temperature should be maintained at 10 ^{0C} ± 2^{0C} throughout the procedure, after partial bunging, loading the vials into a lyophilizer and lyophilizing the said solution to obtain the said drug delivery system.

Rabeprazole disclosed hereinabove is present in a reconstitutable powder composition in a total amount of about 8 % to about 77 %, preferably about 19 % to about 62 %.

The said Mannitol is in the range of 19% to 88%, preferably 30 - 80%.

Benzimidazole compounds and / or their salts are stable in the alkaline pH range and their stability decreases with lowering pH values. Hence, the pH of the composition upon reconstitution should be about 9-11.

The present invention will now be further illustrated by the following non-limiting example.

### Example

Sodium hydroxide was dissolved in Water for Injection (approx- 38 liters) to make 0.01M solution. The pH of the said solution was adjusted above 12.0. Mannitol (600gm) and Rabeprazole sodium (447gm) were added to the above solution, maintaining the pH and making up the volume to 40 liters. The above solution was filtered aseptically through 0.22µ filter paper and 2.0 ml of filtered solution was filled in previously sterilized 10ml vials, the solution temperature should be maintained at 10°C ± 2°C throughout the procedure. After partial bunging, the vials were loaded into the lyophilizer. The lyophilizer shelf temperature was maintained at 5°C ± 2°C during the charging operation.

After the vial loading, the vials were chilled to - 40° C and held at this temperature for 2 hours. At the end of 2 hours, the condenser was chilled to below -40°C. Next, the condenser was vaccumized to less than 200 micron before opening the butterfly valve. After opening the butterfly valve, during first hour, the lyophiliser chamber conditions were allowed to stabilize. Next 4 hours, heating medium temperature was maintained at - 20° C. Next 6 hours, the heating medium temperature was maintained at - 10° C. Next 4 hours the heating medium temperature was maintained at - 5° C.

Next 4 hours, the heating medium temperature maintained at 0°C. Next 1 hour heating medium temperature maintained at 5°C. Next 2 hours heating medium temperature maintained at 10°C. Next 1 hour heating medium temperature maintained at 15°C. Next 1 hour heating medium temperature maintained at 20°C. Next, the heating medium temperature was maintained at 30°C until 4 micron point was reached.

The cycle was terminated after reaching 4 micron end point, butterfly valve was closed and break the vacuum, with sterile filtered Nitrogen. After the batch completion, full stoppering and sealing was carried out.

The resulting formulation contained the following components in the following amounts.

**Rabeprazole Sodium for injection 20mg/vial**

| Ingredients | Specification | Qty per vial | Qty per 300ml (150 vials) | Qty per 20,000 vials |
|---|---|---|---|---|
| Rabeprazole Sodium eq to Rabeprazole | IH | 20 mg | 3.352 gm | 447 gm |
| Mannitol | IP | 30 mg | 4.5 gm | 600 gm |
| Sodium hydroxide | IP | 0.8 mg to adjust pH | 120 mg | 16 gm |
| Water for Injection before lyophilization qs. | IP | 2.0 ml | 300 ml | 40 Its |

The example mentioned above for Rabeprazole and the process for lyophilization, the conditions including pH and pharmaceutically acceptable inactive ingredients used therein are also applicable for other Proton Pump Inhibitors and lyophilized dosage forms thereof.

Stability studies were carried out as per ICH guidelines for the said Rabeprazole composition at accelerated conditions and the results obtained were satisfactory. While the present invention is described above in connection with preferred or illustrative embodiments, these embodiments are not intended to be exhaustive or limiting of the invention.

## Claims

1. A process for the preparation of a stabilised drug delivery system which comprises dissolving sodium hydroxide in water suitable for injection and adjusting the pH of the solution above 12.0, adding mannitol and a benzimidazole compound to the above said solution whilst maintaining the pH of the said solution; making up the solution to a desired volume with water suitable for injection and lyophilizing the said made up solution to obtain in powder form the drug delivery system which is reconstitutable in a parenterally acceptable solvent to form an injectable solution, whilst maintaining the solution at a temperature of 10°C ± 2°C throughout the process prior to lyophilisation.

2. A process according to claim 1 comprising the step of filtering the said solution prior to lyophilisation.

3. A process according to claim 2 wherein the filtering is performed aseptically through 0.22µm filter paper.

4. A process according to claim 3 comprising transferring the said filtered solution to previously sterilized vials.

5. A process according to claim 4 wherein the vials are 10ml vials.

6. A process according to any one of claims 1 to 5 wherein the benzimidazole compound comprises Rabeprazole or one or more of its salts.

7. A process according to any one of claims 1 to 6 wherein one or more pharmaceutically acceptable excipients is included in the system.

8. A process according to any one of claims 1 to 7 wherein the benzimidazole compound is provided in the form of an alkali metal salt or an alkaline earth metal salt.

9. A process according to claim 8 wherein the said alkali metal salt is a sodium salt or a potassium salt.

10. A process according to claim 8 wherein the said alkaline earth metal salt is a calcium salt or a magnesium salt.

11. A process according to any one of claims 1 to 10 comprising reconstituting the drug delivery system in a parenterally acceptable solvent to form an injectable solution, the pH of the said injectable solution being between 9 and 11.

12. A process according to any one of claims 1 to 11 wherein the benzimidazole compound is present in the drug delivery system in an amount of from 8% to 77% by weight thereof.

13. A process according to claim 12 wherein the benzimidazole compound is present in the drug delivery system in an amount of from 19 to 62% by weight thereof.

14. A process according to any one of claims 1 to 13 wherein mannitol is present in the drug delivery system in an amount of from 19% to 88% by weight thereof.

15. A process according to claim 14 wherein mannitol is present in the drug delivery system in an amount of from 30% to 88% by weight thereof.

## Patentansprüche

1. Verfahren zur Herstellung eines stabilisierten Arzneimittelabgabesystems, das Folgendes einschließt: Lösen von Natronlauge in Wasser zur Injektion und Einstellen des pH-Werts der Lösung über 12,0, Hinzugeben von Mannitol und einer Benzimidazolverbindung zu der oben genannten Lösung unter Aufrechterhaltung des pH-Werts der Lösung; Auffüllen des gewünschten Volumens der Lösung mit Wasser zur Injektion und Lyophilisieren der aufgefüllten Lösung, um das Arzneimittelabgabesystem in Pulverform zu erhalten, das in einem parenteral verträglichen Lösungsmittel zum Bilden einer injizierbaren Lösung wieder hergestellt werden kann, unter Aufrechterhaltung der Temperatur der Lösung auf 10°C ± 2°C während des Verfahrens vor der Lyophilisierung.

2. Verfahren nach Anspruch 1, das Folgendes einschließt: Filtern der Lösung vor der Lyophilisierung.

3. Verahren nach Anspruch 2, bei dem das Filtern aseptisch durch 0,22 µm Filterpapier durchgeführt wird.

4. Verfahren nach Anspruch 3, das Folgendes einschließt: Überführen der gefilterten Lösung in vorhergehend sterilisierte Ampullen.

5. Verfahren nach Anspruch 4, bei dem die Ampullen 10 ml Ampullen sind.

6. Verfahren nach einem der Ansprüche 1 bis 5, bei dem die Benzimidazolverbindung Rabeprazol oder ein oder mehrere seiner Salze aufweist.

7. Verfahren nach einem der Ansprüche 1 bis 6, bei dem das System einer oder mehrere pharmazeutisch verträgliche(n) Arzneimittelträge einschließt.

8. Verfahren nach einem der Ansprüche 1 bis 7, bei dein die Benzimidazolverbindung in Form von einem Alkailmetallsalz oder einem Erdalkalimetallsalz vorliegt.

9. Verfahren nach Anspruch 8, bei dem das Alkalimetallsalz ein Natriumsalz oder ein Kaliumsalz darstellt.

10. Verfahren nach Anspruch 8, bei dem das Erdalltalimetallsalz ein Kaliumsalz oder ein Magnesiumsalz darstellt.

11. Verfahren nach einem der Ansprüche 1 bis 10, das Folgendes einschließt: Wiederherstellen des Arzneimittelabgabesystems in einem parenteral verträglichen Lösungsmittel zum Bilden einer injizierbaren Lösung, wobei der pH-Wert der injizierbaren Lösung zwischen 9 und 11 beträgt.

12. Verfahren nach einem der Ansprüche 1 bis 11, bei dem der Anteil der Benzimidazolverbindung in dem Arzneimittelabgabesystem in einer Menge von 8 bis 77 Gewichtsprozent desselben vorliegt.

13. Verfahren nach Anspruch 12, bei dem der Anteil der Benzimidazolverbindung in dem Arzneimittelabgabesystem in einer Menge von 19 bis 62 Gewichtsprozent desselben vorliegt.

14. Verfahren nach einem der Ansprüche 1 bis 13, bei dem der Anteil des Mannitols in dem Arzneimittelabgabesystem in einer Menge von 19 bis 88 Gewichtsprozent desselben vorliegt.

15. Verfahren nach Anspruch 14, bei dem der Anteil des Mannitols in dem Arzneimittelabgabesystem in einer Menge von 30 bis 88 Gewichtsprozent desselben vorliegt.

## Revendications

1. Processus de préparation d'un système d'administration de médicament stabilisé qui comprend les étapes suivantes : dissoudre de l'hydroxyde de sodium dans de l'eau convenant à l'injection et rajuster le pH de la solution pour qu'il soit supérieur à 12,0, ajouter du mannitol et un composé de benzimidazole à ladite solution ci-dessus tout en maintenant le pH de ladite solution ; donner le volume voulu à ladite solution en y ajoutant de l'eau convenant à l'injection et lyophiliser ladite solution préparée pour obtenir en forme de poudre le système d'administration de médicament qui peut être reconstitué dans un solvant pour administration parentérale afin de former une solution injectable, tout en maintenant la solution à une température de 10°C ± 2°C pendant tout le processus préalable à la lyophilisation.

2. Processus conforme à la revendication 1, comprenant l'étape consistant à filtrer ladite solution avant la lyophilisation.

3. Processus conforme à la revendication 2, où le filtrage est exécuté en conditions d'asepsie à travers un papier filtre de 0,22 µm.

4. Processus conforme à la revendication 3, comprenant l'étape consistant à transférer ladite solution filtrée dans des flacons précédemment stérilisés.

5. Processus conforme à la revendication 4, où les flacons sont des flacons de 10 ml.

6. Processus conforme à une quelconque des revendications 1 à 5, où le composé de benzimidazole comprend du rabéprazole ou l'un ou plusieurs de ses sels.

7. Processus conforme à une quelconque des revendications 1 à 6, où l'un ou plusieurs excipients pharmaceutiques acceptables sont inclus dans le système.

8. Processus conforme à une quelconque des revendications 1 à 7, où le composé de benzimidazole est fourni sous la forme d'un sel de métal alcalin ou d'un sel de métal alcalinoterreux.

9. Processus conforme à la revendication 8, où ledit sel de métal alcalin est un sel de sodium ou un sel de potassium.

10. Processus conforme à la revendication 8, où ledit sel de métal alcalinoterreux est un sel de calcium ou un sel de magnésium.

11. Processus conforme à une quelconque des revendications 1 à 10, comprenant l'étape consistant à reconstituer le système d'administration de médicament dans un solvant pour administration parentérale afin de former une solution injectable, le pH de ladite solution injectable étant compris entre 9 et 11.

12. Processus conforme à une quelconque des revendications 1 à 11, où le composé de benzimidazole est présent dans le système d'administration de médicament dans une quantité allant de 8% à 77% en poids de celui-ci.

13. Processus conforme à la revendication 12, où le composé de benzimidazole est présent dans le système d'administration de médicament dans une quantité allant de 19% à 62% en poids de celui-ci.

14. Processus conforme à une quelconque des revendications 1 à 13, où le mannitol est présent dans le système d'administration de médicament dans une quantité allant de 19% à 88% en poids de celui-ci.

15. Processus conforme à la revendication in, où le mannitol est présent dans le système d'administration de médicament dans une quantité allant de 30% à 88% en poids de celui-ci.
